# NEUE EUROPÄISCHE PATENTSCHRIFT

(11) **EP 1 940 766 B2**
(45) Veröffentlichungstag und Bekanntmachung des Hinweises auf die Entscheidung über den Einspruch: **04.09.2019**
(45) Hinweis auf die Patenterteilung: 28.12.2011
(21) Anmeldenummer: 06793087.5
(22) Anmeldetag: 31.08.2006
(51) Int. Cl.: C07C 51/41, C07C 57/04, B01J 14/00, C08F 20/06, C08F 220/06

(54) **NEUTRALISATIONSVERFAHREN**
NEUTRALIZATION PROCESS
PROCEDE DE NEUTRALISATION

(30) Priorität: 07.09.2005 DE 102005042606
(43) Veröffentlichungstag der Anmeldung: 09.07.2008
(73) Patentinhaber: BASF SE, 67056 Ludwigshafen am Rhein (DE)
(72) Erfinder: MARCO, Michael, 69469 Weinheim (DE); WEISMANTEL, Matthias, 63637 Jossgrund (DE); POSSEMIERS, Karl, J., B-2900 Schoten (BE); MEES, Filip, B-2280 Grobbendonk (BE); DE KAEY, Ronny, B-2531 Vremde (BE); FUNK, Rüdiger, 65527 Niedernhausen (DE)
(74) Vertreter: BASF IP Association
(86) Internationale Anmeldenummer: PCT/EP2006/065843
(87) Internationale Veröffentlichungsnummer: WO 2007/028747

(56) Entgegenhaltungen:
- EP-A1- 0 574 260
- EP-A2- 0 372 706
- EP-A2- 0 372 706
- WO-A-03/051415
- WO-A1-03/095410
- WO-A1-2004/094482
- WO-A2-01/09273
- DE-A1- 10 221 203
- JP-A- 2001 098 002
- US-A- 5 397 845
- US-B1- 6 388 000
- "Commercial Processes for the Manufacture of Superabsorbent Polymers"; "Chapter 3" In: Buckholz, F. et al: "Modern Superabsorbent Polymer Technology", 1998, John Wiley & Sons pages 69-84,

## Beschreibung

Die vorliegende Erfindung betrifft ein Neutralisationsverfahren für ethylenisch ungesättigte Carbonsäuren, wobei eine vorneutralisierte Lösung in mindestens zwei Teillösungen aufgeteilt wird und mindestens eine Teillösung nachbehandelt wird, so dass Teillösungen mit unterschiedlichem Neutralisationsgrad und/oder Feststoffgehalt entstehen, sowie eine Vorrichtung zur Durchführung des Verfahrens.

Weitere Ausführungsformen der vorliegenden Erfindung sind den Ansprüchen und der Beschreibung zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure bevorzugt werden. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Herstellung der wasserabsorbierenden Polymere wird beispielsweise in der Monographie "Modern Superabsorbent Polymer Technology", F.L. Buchholz und A.T. Graham, Wiley-VCH, 1998, oder in Ullmann's Encyclopedia of Industrial Chemistry, 6. Auflage, Band 35, Seiten 73 bis 103, beschrieben. Das bevorzugte Herstellungsverfahren ist die Lösungs- oder Gelpolymerisation. Bei dieser Technologie wird zunächst eine Monomermischung hergestellt, die diskontinuierlich neutralisiert und dann in einen Polymerisationsreaktor überführt wird, oder bereits im Polymerisationsreaktor vorgelegt wird. Im sich anschließenden diskontinuierlichen oder kontinuierlichen Verfahren erfolgt die Reaktion zum Polymergel, das im Falle einer gerührten Polymerisation bereits zerkleinert wird. Das Polymergel wird anschließend getrocknet, gemahlen und gesiebt und dann zur weiteren Oberflächenbehandlung transferiert.

Ein kontinuierliches Polymerisationsverfahren liegt beispielsweise der WO 01/38402 zugrunde, wobei die wässrige Monomerlösung kontinuierlich einem Mischkneter mit mindestens zwei achsparallel rotierenden Wellen zugeführt wird.

Kontinuierliche Gelpolymerisationen sind weiterhin bekannt aus WO 03/004237, WO 03/022896 und WO 01/016197.

Sowohl bei der kontinuierlichen als auch bei der diskontinuierlichen Polymerisation wird die Acrylsäure im Falle der Vorneutralisation diskontinuierlich neutralisiert. Typischerweise werden im Polymerisationsreaktor im Falle der Lösungspolymerisation die Edukte Acrylsäure, Wasser, optionale Comonomere und Natronlauge diskontinuierlich dosiert und vermischt. In diesem Schritt wird weitestgehend der restliche Polymerisationsverlauf und auch die zu erwartenden Polymereigenschaften festgelegt. Der Vernetzungsgrad des Basispolymers sowie der Neutralisationsgrad werden typischerweise in diesem Schritt bestimmt. Der Neutralisationsgrad der Monomeren liegt zwischen 0 und 80 mol-%. Im Falle der sauren Polymerisation wird das erhaltene Polymergel üblicherweise zu 50 bis 80 mol-%, vorzugsweise zu 60 bis 75 mol-% nachneutralisiert, indem Natriumhydroxid- oder Natriumcarbonatlösung zum sauren Polymergel zugegeben und eingearbeitet wird.

Neutralisationsverfahren werden beispielsweise in EP-A 0 372 706, EP-A 0 574 260 und WO 03/051415 beschrieben.

EP-A 0 372 706 beschreibt ein dreistufiges Neutralisationsverfahren, bei dem in einer ersten Stufe Acrylsäure und Natronlauge gleichzeitig dosiert werden, so dass ein Neutralisationsgrad von 75 bis 100 mol-% eingehalten wird, in einer zweiten Stufe der Neutralisationsgrad auf 100, 1 bis 110 mol-% angehoben wird um in der eingesetzten Acrylsäure als Verunreinigung enthaltene Diacrylsäure zu hydrolysieren, und in einer dritten Stufe durch Zusatz von weiterer Acrylsäure ein Neutralisationsgrad von 20 bis 100 mol-% eingestellt wird.

EP-A 0 574 260 offenbart auf Seite 7, Zeilen 38 bis 41, dass bei der Neutralisation vorteilhaft Natronlauge vorgelegt und anschließend Acrylsäure unter Kühlung zugesetzt wird.

WO 03/051415 lehrt ein Verfahren zur Herstellung wasserabsorbierender Polymere, bei dem die Monomerlösung eine Mindesttemperatur von 40°C aufweist.

Es ist bekannt, dass sich die Reaktivität der Acrylsäure sehr stark von der ihrer Salze unterscheidet, weshalb auch der Polymerisationsverlauf stark abhängig ist vom pH-Wert, bei dem er stattfindet. In einer anschaulichen Darstellung in der Monographie "Modern Superabsorbent Polymer Technology", F.L. Buchholz und A.T. Graham, Wiley-VCH, 1998, oder in Ullmann's Encyclopedia of Industrial Chemistry, 6. Auflage, Band 35, Seite 35, ist die Polymerisationsgeschwindigkeit als Funktion des pH-Wertes aufgetragen. Demnach durchläuft die Polymerisationsgeschwindigkeit ein Minimum bei einem pH-Wert von 6 bis 7. Dies entspricht allerdings dem pH-Wert, der in aller Regel bei den Verkaufsprodukten gewünscht ist. Erklärt wird dieses Verhalten dadurch, dass es zwischen dem geladenen Monomeren in Salzform und der wachsenden Radikalkette zu elektrostatischen Abstoßungsreaktionen kommt, die im Falle der weitestgehend undissoziierten Acrylsäure nicht vorliegen, was zur Verlangsamung des Reaktionsverlaufs führt.

Es ist auch bekannt, dass unneutralisierte Acrylsäure leichter polymerisiert werden kann als vorneutralisierte Systeme. Diese Differenz wird jedoch bei steigender Monomerkonzentration verringert, vor allem deswegen, weil eine höhere Monomerkonzentration die Dissoziation der Acrylsäuresalze unterdrückt.

Um all diesen reaktionstechnischen Details Rechnung zu tragen, werden bei der Reaktionsführung üblicherweise Kompromisse eingegangen.

Generell wird der Neutralisationsgrad der Acrylsäure bereits vor dem Eintritt in die kontinuierliche Polymerisation eingestellt. Die Neutralisation erfolgt diskontinuierlich. Die diskontinuierliche Neutralisation hat den Vorteil, dass die Dosierung von Acrylsäure und/oder Natronlauge temperaturgeregelt erfolgen kann. Damit werden Überhitzungen und unerwünschte Polymerisationen in dem Ansatzbehälter vermieden. Der Neutralisationsgrad wird entsprechend den Polymerisationsbedingungen und dem erwünschten Absorptionsprofil gewählt und wahlweise in einer nachgeschalteten Neutralisation, die zumeist am Polymergel erfolgt, korrigiert.

Üblicherweise verfügt jede Produktionsanlage für wasserabsorbierende Polymerpartikel über eine separate Neutralisation. Dadurch können auf den einzelnen Produktionsanlagen Produkte mit unterschiedlichen Eigenschaften hergestellt werden.

Aufgabe der vorliegenden Erfindung war die Bereitstellung eines verbesserten Neutralisationsverfahrens, wobei auf vereinfachte Weise neutralisierte Lösungen mit unterschiedlichen Neutralisationsgrad und/oder Feststoffgehalt hergestellt werden können.

Gelöst wurde die Aufgabe durch ein Neutralisationsverfahren, wobei mindestens eine ethylenisch ungesättigte Carbonsäure zumindest teilweise mit einer Base, wahlweise unter Zusatz von Wasser, vorneutralisiert wird, dadurch gekennzeichnet, dass die Vorneutralisation kontinuierlich durchgeführt wird, dass die vorneutralisierte Lösung teilweise in die Vorneutralisation rückgeführt wird und dass die vorneutralisierte Lösung in mindestens zwei Teillösungen aufgeteilt wird, wobei mindestens eine Teillösung nachbehandelt wird, so dass Teillösungen mit unterschiedlichem Neutralisationsgrad und/oder Feststoffgehalt entstehen, und wobei der Neutralisationsgrag der vorneutralisierten Lösung durch Zugabe von Base erhöht wird oder der Neutralisationsgrad der vorneutralisierten Lösung durch Zugabe von ethylenisch ungesättigter Carbonsäure gesenkt wird und/oder der Feststoffgehalt der vorneutralisierten Lösung durch Zusatz von Wasser gesenkt wird.

Die vorneutralisierte Lösung wird in zwei, drei, vier, fünf, sechs, sieben oder mehr Teillösungen aufgeteilt, vorzugsweise in zwei oder drei Teillösungen.

Erfindungsgemäß besteht für mindestens eine Teillösung die Möglichkeit den Neutralisationsgrad und/oder den Feststoffgehalt der vorneutralisierten Lösung zu verändern, vorzugsweise für alle Teillösungen.

Der Neutralisationsgrad wird durch Zusatz von Base erhöht und durch Zusatz von ethylenisch ungesättigter Carbonsäuren gesenkt.

Der Feststoffgehalt wird durch Zusatz von Wasser gesenkt.

Vorteilhaft ist der Neutralisationsgrad der vorneutralisierten Lösung nicht höher als der höchste Neutralisati-onsgrad der nachbehandelten Teillösungen. Dadurch kann bei der Nachbehandlung auf die sicherheitstechnisch auf-wendigere Dosierung von ethylenisch ungesättigter Carbonsäure verzichtet werden.

Wird neben Wasser bei der Nachbehandlung einer Teillösung zusätzlich ethylenisch ungesättigte Carbonsäure oder Base dosiert, so wird Wasser vorzugsweise zuerst dosiert.

Wird eine Teillösung mit Base und Wasser nachbehandelt, so werden Wasser und Base vorzugsweise vorgemischt. In diesem Fall kann die dabei freiwerdende Lösungswärme bereits vor der Nachbehandlung, beispielsweise mittels geeigneter Wärmeaustauscher, abgeführt werden.

Nach dem erfindungsgemäßen Verfahren können beispielsweise zwei Teillösungen 1 und 2 mit unterschied-lichem Neutralisationsgrad und gleichem Feststoffgehalt erzeugt werden. Dazu wird durch Vorneutralisation eine vor-neutralisierte Lösung hergestellt, aus der wahlweise durch Zusatz von Wasser die gewünschte Teillösung 1 und durch Zusatz von 50gew.-%iger Natronlauge und wahlweise durch Zusatz von Wasser die gewünschte Teillösung 2 hergestellt werden kann. Selbstverständlich muss berücksichtigt werden, dass über die Natronlauge auch Wasser eingetragen wird.

Nach dem erfindungsgemäßen Verfahren können beispielsweise zwei Teillösungen 1 und 2 mit gleichem Neutralisationsgrad und unterschiedlichem Feststoffgehalt erzeugt werden. Dazu wird durch Vorneutralisation eine vor-neutralisierte Lösung hergestellt, die der gewünschten Teillösung 1 entspricht, und aus der durch Zusatz von Wasser die gewünschte Teillösung 2 hergestellt werden kann.

Die Berechnungen werden vorzugsweise durch ein Computerprogramm durchgeführt, beispielsweise durch ein handelsübliches Prozeßleitsystem.

Vorzugsweise werden ethylenisch ungesättigte Carbonsäuren, wie Acrylsäure, Methacrylsäure, Maleinsäure, Fumarsäure und Itaconsäure, eingesetzt. Acrylsäure ist besonders bevorzugt.

Die Temperatur der ethylenisch ungesättigte Carbonsäure beträgt üblicherweise von 0 bis 40°C, vorzugsweise von 5 bis 35°C, besonders bevorzugt von 10 bis 30°C, ganz besonders bevorzugt von 15 bis 25°C, wobei auf einen ausreichenden Abstand zu Schmelzpunkt zu achten ist. Bei Verwendung von Acrylsäure sollte eine Temperatur von 15°C auf keinen Fall unterschritten werden.

Als Base ist wässriges Alkali bevorzugt. Wässriges Alkali sind alle alkalisch reagierenden wässrigen Lösungen, d.h. wässrige Lösungen mit einem pH-Wert von mindestens 8, vorzugsweise mindestens 10, besonders bevorzugt mindestens 12, ganz besonders bevorzugt mindestens 14.

Die im wässrigen Neutralisationsmittel einsetzbaren alkalischen Salze sind vorzugsweise Alkalimetallhydroxide, Alkalimetalloxide, Alkalimetallcarbonate und Alkalimetallhydrogencarbonate sowie deren Mischungen. Statt Alkalimetallsalzen können auch Ammoniumsalze verwendet werden. Natrium und Kalium als Alkalimetalle sind besonders bevorzugt, ganz besonders bevorzugt jedoch Natriumhydroxid, Natriumcarbonat oder Natriumhydrogencarbonat sowie deren Mischungen. Üblicherweise beträgt der Gehalt des alkalischen Salzes im wässrigen Alkali mindestens 10 Gew.-%, vorzugsweise mindestens 20 Gew.-%, bevorzugt mindestens 30 Gew.-%, ganz besonders bevorzugt mindestens 40 Gew.-%.

Die Temperatur des wässrigen Alkali beträgt üblicherweise von 0 bis 45°C, vorzugsweise von 5 bis 40°C, besonders bevorzugt von 10 bis 35°C, ganz besonders bevorzugt von 15 bis 30°C, wobei Übersättigungen und damit Ausfällungen zu vermeiden sind.

Beträgt der Alkaligehalt des wässrigen Alkali mindestens 25 Gew.-%, so sind höhere Temperaturen vorteilhaft, üblicherweise von 10 bis 60°C, vorzugsweise von 20 bis 55°C, besonders bevorzugt von 30 bis 50°C, ganz besonders bevorzugt von 40 bis 45°C.

Das Verhältnis von ethylenisch ungesättigter Carbonsäure zu Base wird üblicherweise so gewählt, dass der Neutralisationsgrad der ethylenisch ungesättigter Carbonsäure nach der Neutralisation vorzugsweise von 25 bis 85 mol-%, bevorzugt von 27 bis 80 mol-%, besonders bevorzugt von 27 bis 30 mol-% oder 40 bis 75 mol-%, beträgt.

Der Neutralisationsgrad ist das Molverhältnis von neutralisierter ethylenisch ungesättigter Carbonsäure nach der Neutralisation zur Gesamtmenge eingesetzter ethylenisch ungesättigter Carbonsäure vor der Neutralisation.

Der Neutralisationsgrad nach der Vorneutralisation liegt üblicherweise 0 bis 50 mol-%, vorzugsweise 1 bis 40 mol-%, besonders bevorzugt 2 bis 30 mol-%, ganz besonders bevorzugt 5 bis 20 mol-%, niedriger als der höchste Neutralisationsgrad aller Teillösungen.

Die Vorneutralisation wird kontinuierlich durchgeführt und/ die Nachbehandlung wird worzugsweise kontinuierlich durchgeführt. Besonders bevorzugt werden beide Verfahrensschritte kontinuierlich durchgeführt. Die bedeutet, dass dem Neutralisationsbereich ethylenisch ungesättigte Carbonsäure und/oder Base zugeführ vird und dem Neutralisationsbereich gleichzeitig neutralisierte Lösung entnommen wird. An- und Abfahrvorgänge des kontinuierlichen Neutralisationsverfahrens sind hiervon selbstverständlich ausgenommen.

Der Neutralisationsbereich ist der Bereich, in dem die Neutralisation weitgehend stattfindet, d.h. der Bereich in dem ethylenisch ungesättigte Carbonsäure und Base unter Salzbildung reagieren (Neutralisation).

Die Neutralisation ist weitgehend abgeschlossen, wenn der Umsatz der Neutralisation mindestens 90 mol-%, vorzugsweise mindestens 95 mol-%, bevorzugt mindestens 98 mol-%, ganz besonders bevorzugt mindestens 99 mol-%, beträgt. Der Umsatz kann leicht über die freigesetzte Neutralisationswärme durch Vergleich mit der theoretischen Wärmetönung ermittelt werden.

Die kontinuierliche Vorneutralisation wird so durchgeführt, dass die Temperatur der neutralisierten Lösung vorzugsweise weniger als 60°C, bevorzugt weniger als 50°C, besonders bevorzugt weniger als 40°C, ganz besonders bevorzugt weniger als 30°C, beträgt, wobei die Temperatur die Durchschnittstemperatur nach der Vorneutralisation ist, d.h. die Mitteltemperatur nach vollständiger Wärmetönung.

Zusätzlich kann die vorneutralisierte Lösung mit Wasser verdünnt werden. Über die Verdünnung mit Wasser kann der Feststoffgehalt der vorneutralisierten Lösung eingestellt werden. Der Feststoffgehalt ist die Summe der Gewichtanteile an neutralisierter ethylenisch ungesättigter Carbonsäure und wahlweise überschüssiger ethylenisch ungesättigter Carbonsäure oder überschüssiger Base. Der Feststoffgehalt der vorneutralisierten Lösung beträgt typischerweise von 10 bis 80 Gew.-%, vorzugsweise von 20 bis 70 Gew.-%, besonders bevorzugt von 30 bis 60 Gew.-%.

Die Temperatur des Wassers beträgt üblicherweise von über 0 bis 40°C, vorzugsweise von 5 bis 35°C, besonders bevorzugt von 10 bis 30°C, ganz besonders bevorzugt von 15 bis 25°C.

Vorteilhaft wird die vorneutralisierte Lösung gekühlt, wobei die zur Kühlung einsetzbaren Wärmeaustauscher keiner Beschränkung unterliegen. Die vorneutralisierte Lösung wird auf eine Temperatur von vorzugsweise weniger als 50°C, bevorzugt weniger als 40°C, besonders bevorzugt weniger als 30°C, ganz besonders bevorzugt weniger als 20°C, gekühlt. Die Kühlung sollte möglichst nah an der Vorneutralisation sein, da so hohe Verweilzeiten der vorneutralisierten Lösung bei hohen Temperaturen vermieden werden können.

Vorzugsweise werden Wasser und Base vorgemischt. In diesem Fall kann die dabei freiwerdende Lösungswärme bereits vor der Neutralisation, beispielsweise mittels geeigneter Wärmeaustauscher, abgeführt werden.

In einer besonders bevorzugten Ausführungsform der vorliegenden Erfindung wird ein Teil der vorneutralisierten Lösung gekühlt in die Neutralisation rückgeführt.

Durch die Rückführung kann die Neutralisationswärme und die Lösungswärme besser verteilt und Temperaturspitzen (Peaktemperatur) in der Mischung niedrig gehalten werden. Der Anteil rückgeführter vorneutralisierter Lösung beträgt üblicherweise von 25 bis 99%, vorzugsweise von 33 bis 98%, besonders bevorzugt von 50 bis 95%, ganz besonders bevorzugt von 80 bis 90%, jeweils bezogen auf die vorneutralisierte Lösung.

Die ethylenisch ungesättigte Carbonsäure, die Base und wahlweise das Wasser können an beliebigen Stellen in die rückgeführte vorneutralisierte Lösung dosiert werden. Vorzugsweise werden die Flüssigkeiten nacheinander dosiert, besonders bevorzugt Base und ethylenisch ungesättigte Carbonsäure nacheinander, ganz besonders bevorzugt Wasser, Base und ethylenisch ungesättigte Carbonsäure nacheinander.

Vorteilhaft wird mindestens eines der Edukte über zwei oder mehr getrennte Zugabestellen dosiert.

Beispielsweise können die Edukte über zwei, drei, vier, fünf oder sechs Zugabestellen dosiert werden, wobei die Zugabestellen vorzugsweise so angeordnet sind, dass sie eine gemeinsame Achse aufweisen (für zwei Zugabestellen) oder einen symmetrischen Stern bilden (für mindestens drei Zugabestellen) und die Achse bzw. Stern senkrecht zur Flussrichtung der neutralisierten Lösung befindet (Mehrfachzugabestellen). Besonders vorteilhaft wird die Base dosiert, wenn zwei, drei oder vier Mehrfachzugabestellen hintereinander angeordnet werden.

Das Aufteilen in mehrere Zugabestellen bewirkt eine gleichmäßigere Durchmischung und niedrigere Temperaturspitzen, was die Gefahr unerwünschter Polymerisation vermindert.

In einer weiteren Ausführungsform werden Wasser und Base so dosiert, dass das Wasser die Base beim Eintritt in die Vorneutralisation umhüllt. Dazu können beispielsweise zwei ineinander gesteckte Rohre verwendet werden, wobei die Base über das Innenrohr und das Wasser über den Ringspalt zwischen innerem und äußeren Rohr dosiert werden.

Vorteilhaft enthält die Vorneutralisation einen zusätzlichen Behälter als Puffergefäß.

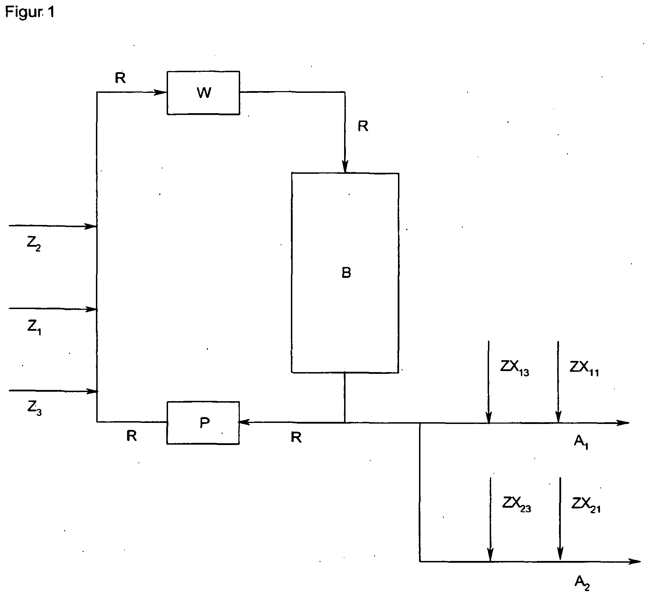

Eine beispielhafte erfindungsgemäße Neutralisation zeigt Figur 1, wobei die Bezugszeichen folgende Bedeutungen haben:

| | |
|---|---|
| Z₁ bis Z₃ | Zuführungen für Edukte 1 bis 3 |
| ZX₁₁, ZX₂₁, ZX₁₃ und ZX₂₃ | Zuführungen für Edukte 1 und 3 |
| A₁ und A₂ | Ableitungen |
| B | Behälter |
| P | Pumpe |
| R | Ringleitung |
| W | Wärmeaustauscher |

Mittels einer Pumpe P wird vorneutralisierte Lösung teilweise über die Ringleitung R rückgeführt. Der Rest der vorneutralisierten Lösung wird über die Ableitungen A₁ und A₂ der weiteren Verwendung zugeführt. Der Behälter B dient als Puffer. Über die Zuleitung Z₁ wird vorzugsweise 50gew.-%ige Natronlauge, über die Zuleitung Z₂ wird vorzugsweise Acrylsäure und über die Zuleitung Z₃ wird vorzugsweise Wasser dosiert.

Die vorneutralisierten Lösungen, die über die Ableitungen A₁ und A₂ abgeführt wird, können über die Zuleitungen ZX₁₁, ZX₂₁, ZX₁₃ und ZX₂₃ nachbehandelt werden. Über die Zuleitungen ZX₁₁ und ZX₂₁ wird vorzugsweise 50 gew.-%ige Natronlauge und über die Zuleitungen ZX₁₃ und ZX₂₃ wird vorzugsweise Wasser dosiert.

Damit die Edukte möglichst intensiv in die rückgeführte vorneutralisierte Lösung eingemischt werden, sollte die Strömung an der Einmischstelle möglichst turbulent sein. Die Einmischstelle ist der Ort, wo das jeweilige Edukt auf die rückgeführte vorneutralisierte Lösung trifft.

In einer bevorzugten Ausführungsform der vorliegenden Erfindung wird mindestens eines der Edukte der Vorneutralisation in ein, vorzugsweise werden alle Edukte der Vorneutralisation in ein, besonders bevorzugt werden alle Edukte der Vorneutralisation in ein gemeinsames, Venturi-Rohr dosiert.

In einer bevorzugten Ausführungsform der vorliegenden Erfindung wird mindestens eines der Edukte der Nachbehandlung in ein, vorzugsweise werden alle Edukte der Nachbehandlung in ein, besonders bevorzugt werden alle Edukte der Nachbehandlung in ein gemeinsames, Venturi-Rohr dosiert.

Ein Venturi-Rohr ist eine in ihrer Länge begrenzte Rohrverengung, in der Druckverlust weitgehend reversibel in kinetische Energie umgewandelt wird. Dazu wird der Querschnitt F₁ auf der Strecke L₁ auf den Querschnitt F₂ vermindert, der Querschnitt F₂ wird auf der Strecke L₂ konstant gehalten und anschließend wird der Querschnitt F₂ auf der Strecke L₃ wieder auf den Querschnitt F₁ geweitet. Dabei ist der Querschnitt F₁ größer als der Querschnitt F₂ und die Länge L₃ größer als die Länge L₁.

Die Dosierung der Edukte für die Neutralisation erfolgt vorzugsweise im Bereich der Strecke L₂ mit dem Querschnitt F₂.

Die optimale Auslegung eines Venturi-Rohrs ist dem Fachmann an sich bekannt. Vorzugsweise wird das Venturi-Rohr so ausgelegt, dass der Druck im Bereich der Strecke L2 weniger als der Umgebungsdruck beträgt (Saugförderung) und/oder das die Strömung im Bereich der Strecke L₂ turbulent ist, wobei die Reynolds-Zahl mindestens 1000, vorzugsweise mindestens 2000, besonders bevorzugt mindestens 3000, ganz besonders bevorzugt mindestens 4000, und üblicherweise weniger als 10.000.000 betragen sollte.

Das erfindungsgemäße Verfahren ist hervorragend dafür geeignet zwei oder mehr Produktionsanlagen aus einer gemeinsamen Neutralisation zu versorgen. Das erfindungsgemäße Verfahren ermöglicht auf einfache Weise die individuelle Anpassung der Lösung an jede angeschlossene Produktion.

Die Hauptmenge der Lösungs- und Neutralisationswärme kann bereits in der gemeinsamen Vorneutralisation abgeführt werden. Damit fallen die hohen Aufwendungen, insbesondere für die sicherheitstechnische Ausstattung nur einmal statt mehrfach an. Sind die geforderten Unterschiede bei Neutralisationsgrad gering, so kann auf eine Kühlung bei der Nachbehandlung verzichtet werden. Ebenso ist in diesem Fall eine geringere Dosiergenauigkeit bei der Nachbehandlung eher tolerierbar, da relative Abweichungen insgesamt weniger stark ins Gewicht fallen.

Ein weiterer Gegenstand der vorliegenden Erfindung ist ein Verfahren zur Herstellung wasserabsorbierender Polymere, in dem eine gemäß dem erfindungsgemäßen Neutralisationsverfahren hergestellte neutralisierte Lösungen als Monomerlösungen verwendet werden.

Vorzugsweise wird das erfindungsgemäße Neutralisationsverfahren mit einem kontinuierlichen Polymerisationsverfahren kombiniert, wobei vorzugsweise alle Verfahrensschritte, wie Neutralisation, Polymerisieren, Trocknen, Mahlen, Sieben, Nachvernetzen, Sieben, kontinuierlich durchgeführt werden.

Die wasserabsorbierenden Polymere werden beispielsweise durch Polymerisation einer Monomerlösung, enthaltend
a) mindestens eine ethylenisch ungesättigtes Carbonsäure,
b) mindestens einen Vernetzer,
c) wahlweise ein oder mehrere mit dem Monomeren a) copolymerisierbare ethylenisch und/oder allylisch ungesättigte Monomere und
d) wahlweise ein oder mehrere wasserlösliche Polymere, auf die die Monomere a), b) und ggf. c) zumindest teilweise aufgepfropft werden können,
erhalten.

Geeignete ethylenisch ungesättigte Carbonsäuren a) sind beispielseweise Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden 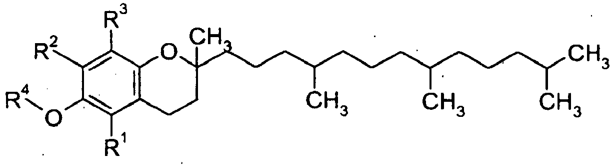 wobei R¹ Wasserstoff oder Methyl, R² Wasserstoff oder Methyl, R³ Wasserstoff oder Methyl und R⁴ Wasserstoff oder ein Säurerest mit 1 bis 20 Kohlenstoffatomen bedeutet.

Bevorzugte Reste für R⁴ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit R¹ = R² = R³ = Methyl, insbesondere racemisches alpha-Tocopherol. R¹ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A 0 530 438 beschrieben, Di- und Triacrylate, wie in EP-A 0 547 847, EP-A 0 559 476, EP-A 0 632 068, WO 93/21237, WO 03/104299, WO 03/104300, WO 03/104301 und DE-A 103 31 450 beschrieben, gemischte Acrylate, die neben Acrylatgruppen weitere ethylenisch ungesättigte Gruppen enthalten, wie in DE-A 103 31 456 und WO 04/013064 beschrieben, oder Vernetzermischungen, wie beispielsweise in DE-A 195 43 368, DE-A 196 46 484, WO 90/15830 und WO 02/32962 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A 0 343 427 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 15-fach ethoxylierten Glyzerins, des 3- bis 15-fach ethoxylierten Trimethylolpropans, des 3- bis 15-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO 03/104301 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3- bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Die Menge der Vernetzer b) beträgt vorzugsweise 0,01 bis 1 Gew.-%, besonders bevorzugt 0,05 bis 0,5 Gew.-%, ganz besonders bevorzugt 0,1 bis 0,3 Gew.-%, jeweils bezogen auf das Monomer a).

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die bevorzugten Polymerisationsinhibitoren benötigen für eine optimale Wirkung gelösten Sauerstoff. Üblicherweise werden die Monomerlösungen vor der Polymerisation weitgehend von Sauerstoff befreit (Inertisierung), beispielsweise mittels Durchströmen mit einem inerten Gas, vorzugsweise Stickstoff. Dadurch werden die Polymerisationsinhibitoren in ihrer Wirkung deutlich abgeschwächt. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Herstellung eines geeigneten Grundpolymers sowie weitere geeignete hydrophile ethylenisch ungesättigte Monomere d) werden in DE-A 199 41 423, EP-A 0 686 650, WO 01/45758 und WO 03/104300 beschrieben.

Wasserabsorbierende Polymere werden üblicherweise durch Polymerisation einer wässrigen Monomerlösung und wahlweise einer anschließenden Zerkleinerung des Hydrogels erhalten. Geeignete Herstellverfahren sind in der Literatur beschrieben. Wasserabsorbierende Polymere können beispielsweise erhalten werden durch
- Gelpolymerisation im Batchverfahren bzw. Rohrreaktor und anschließender Zerkleinerung im Fleischwolf, Extruder oder Kneter (EP-A 0 445 619, DE-A 198 46 413)
- Polymerisation im Kneter, wobei durch beispielsweise gegenläufige Rührwellen kontinuierlich zerkleinert wird, (WO 01/38402)
- Polymerisation auf dem Band und anschließende Zerkleinerung im Fleischwolf, Extruder oder Kneter (DE-A 38 25 366, US 6,241,928)
- Emulsionspolymerisation, wobei bereits Perlpolymerisate relativ enger Gelgrößenverteilung anfallen (EP-A 0 457 660)
- In-situ Polymerisation einer Gewebeschicht, die zumeist im kontinuierlichen Betrieb zuvor mit wässriger Monomerlösung besprüht und anschließend einer Photopolymerisation unterworfen wurde (WO 02/94328, WO 02/94329)

Die Umsetzung wird vorzugsweise in einem Kneter, wie beispielsweise in WO 01/38402 beschrieben, oder auf einem Bandreaktor, wie beispielsweise in EP-A 0 955 086 beschrieben, durchgeführt.

Die Neutralisation kann auch teilweise nach der Polymerisation auf der Stufe des Hydrogels durchgeführt werden. Es ist daher möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Hydrogels eingestellt wird. Die Monomerlösung kann durch Einmischen des Neutralisationsmittels neutralisiert werden. Das Hydrogel kann mechanisch zerkleinert werden, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden. Die Neutralisation der Monomerlösung auf den Endneutralisationsgrad ist bevorzugt.

Das neutralisierte Hydrogel wird dann mit einem Band- oder Walzentrockner getrocknet bis der Restfeuchtegehalt vorzugsweise unter 15 Gew.-%, insbesondere unter 10 Gew.-% liegt, wobei der Wassergehalt gemäß der von der EDANA (European Disposables and Nonwovens Association) empfohlenen Testmethode Nr. 430.2-02 "Moisture content" bestimmt wird. Wahlweise kann zur Trocknung aber auch ein Wirbelbetttrockner oder ein beheizte Pflugscharmischer verwendet werden. Um besonders weiße Produkte zu erhalten, ist es vorteilhaft bei der Trocknung dieses Gels einen schnellen Abtransport des verdampfenden Wassers sicherzustellen. Dazu ist die Trocknertemperatur zu optimieren, die Luftzu- und -abführung muss kontrolliert erfolgen, und es ist in jedem Fall auf ausreichende Belüftung zu achten. Die Trocknung ist naturgemäß umso einfacher und das Produkt umso weißer, je höher der Feststoffgehalt des Gels ist. Bevorzugt liegt der Feststoffgehalt des Gels vor der Trocknung daher zwischen 30 und 80 Gew.-%. Besonders vorteilhaft ist die Belüftung des Trockners mit Stickstoff oder einem anderen nicht-oxidierenden Inertgas. Wahlweise kann aber auch einfach nur der Partialdruck des Sauerstoffs während der Trocknung abgesenkt werden, um oxidative Vergilbungsvorgänge zu verhindern. Im Regelfall führt aber auch eine ausreichende Belüftung und Abführung des Wasserdampfes zu einem noch akzeptablen Produkt. Vorteilhaft hinsichtlich Farbe und Produktqualität ist in der Regel eine möglichst kurze Trocknungszeit.

Das getrocknete Hydrogel wird vorzugsweise gemahlen und gesiebt, wobei zur Mahlung üblicherweise Walzenstühle, Stiftmühlen oder Schwingmühlen eingesetzt werden können. Die Partikelgröße des gesiebten, trockenen Hydrogels beträgt vorzugsweise unter 1000 µm, besonders bevorzugt unter 900 µm, ganz besonders bevorzugt unter 800 µm, und vorzugsweise über 100 µm, besonders bevorzugt über 150 µm, ganz besonders bevorzugt über 200 µm.

Ganz besonders bevorzugt ist eine Partikelgröße (Siebschnitt) von 106 bis 850 µm. Die Partikelgröße wird gemäß der von der EDANA (European Disposables and Nonwovens Association) empfohlenen Testmethode Nr. 420.2-02 "Particle size distribution" bestimmt.

Die Grundpolymere werden vorzugsweise anschließend oberflächennachvernetzt. Hierzu geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A 0 083 022, EP-A 0 543 303 und EP-A 0 937 736 beschrieben, di- oder polyfunktionelle Alkohole, wie in DE-C 33 14 019, DE-C 35 23 617 und EP-A 0 450 922 beschrieben, oder β-Hydroxyalkylamide, wie in DE-A 102 04 938 und US 6,239,230 beschrieben.

Des weiteren sind in DE-A 40 20 780 zyklische Karbonate, in DE-A 198 07 502 2-Oxazolidon und dessen Derivate, wie 2-Hydroxyethyl-2-oxazolidon, in DE-A 198 07 992 Bis- und Poly-2-oxazolidinone, in DE-A 198 54 573 2-Oxotetrahydro-1,3-oxazin und dessen Derivate, in DE-A 198 54 574 N-Acyl-2-Oxazolidone, in DE-A 102 04 937 zyklische Harnstoffe, in DE-A 103 34 584 bizyklische Amidacetale, in EP-A 1 199 327 Oxetane und zyklische Harnstoffe und in WO 03/031482 Morpholin-2,3-dion und dessen Derivate als geeignete Oberflächennachvernetzer beschrieben.

Die Nachvernetzung wird üblicherweise so durchgeführt, dass eine Lösung des O-berflächennachvernetzers auf das Hydrogel oder das trockene Grundpolymerpulver aufgesprüht wird. Im Anschluss an das Aufsprühen wird das Polymerpulver thermisch getrocknet, wobei die Vernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige®-Mischer, Bepex®-Mischer, Nauta®-Mischer, Processall®-Mischer und Schugi®-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und Nara®-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 50 bis 250°C, bevorzugt bei 50 bis 200°C, und besonders bevorzugt bei 50 bis 150°C. Die bevorzugte Verweilzeit bei dieser Temperatur im Reaktionsmischer oder Trockner beträgt unter 30 Minuten, besonders bevorzugt unter 10 Minuten.

Ein weiterer Gegenstand der vorliegenden Erfindung ist eine Vorrichtung zur Durchführung des erfindungsgemäßen Neutralisationsverfahrens, umfassend
i) eine Ringleitung R,
ii) mindestens eine erste Zuleitung Z₁ in die Ringleitung R,
iii) mindestens eine zweite Zuleitung Z₂ in die Ringleitung R,
iv) mindestens einen Wärmeaustauscher W in der Ringleitung R, wobei sich der Wärmeaustauscher W in Flussrichtung hinter den Zuleitungen Z₁ und Z₂ befindet,
v) mindestens zwei Ableitungen Aₙ aus der Ringleitung R, wobei sich die Ableitungen Aₙ in Flussrichtung hinter dem Wärmeaustauscher W befinden,
vi) mindestens eine Zuleitung ZXₙ₁ und/oder ZXₙ₃ in mindestens einer Ableitung Aₙ,
vii) eine Pumpe P und
viii) wahlweise einen Behälter B zwischen dem Wärmeaustauscher W und der Ableitungen Aₙ.
wobei mindestens eine erste Zuleitung Z₁ bedeutet, dass das Edukt 1, beispielsweise Natronlauge, über eine oder mehrere Zuleitungen Z₁ zugeführt wird, mindestens eine zweite Zuleitung Z₂ bedeutet, dass das Edukt 2, beispielsweise Acrylsäure, über eine oder mehrere Zuleitungen Z₂ zugeführt wird, mindestens eine Zuleitung Zₙ₁ bedeutet, dass das Edukt 1, beispielsweise Natronlauge, über eine oder mehrere Zuleitungen Zₙ₁ zugeführt wird und mindestens eine Zuleitung Zₙ₃ bedeutet, dass das Edukt 3, beispielsweise Wasser, über eine oder mehrere Zuleitungen Zₙ₃ zugeführt wird sowie n die Laufvariable der Ableitungen A darstellt, wobei n gleich 2, 3, 4, 5, 6, 7 oder mehr sein kann.

n ist vorzugsweise 2 oder 3.

Der Ringleitungsquerschnitt Q beträgt von vorzugsweise von 20 bis 2000 cm², besonders bevorzugt von 80 bis 700 cm², ganz besonders bevorzugt von 200 bis 500 cm². Die Ringleitung R hat vorzugsweise einen kreisrunden Querschnitt.

Die Summe der Zuleitungen Z₁ hat einen Querschnitt vorzugsweise von 1,5 bis 100 cm², besonders bevorzugt von 6 bis 35 cm², ganz besonders bevorzugt von 15 bis 25 cm². Die Zuleitungen Z₁ haben vorzugsweise einen kreisrunden Querschnitt.

Die Summe der Zuleitungen Z₂ hat einen Querschnitt vorzugsweise von 1,5 bis 100 cm², besonders bevorzugt von 6 bis 35 cm², ganz besonders bevorzugt von 15 bis 25 cm². Die Zuleitungen Z₂ haben vorzugsweise einen kreisrunden Querschnitt.

Die Pumpe P hat eine Förderleistung von vorzugsweise 1 bis 1000 t/h, besonders bevorzugt von 10 bis 700 t/h, ganz besonders bevorzugt von 100 bis 500 t/h.

Der Behälter B hat ein Volumen von vorzugsweise 1 bis 100 m³, besonders bevorzugt von 10 bis 100 m³, ganz besonders bevorzugt von 20 bis 50 m³.

Die Zuleitungen Z₁ und Z₂ sind vorzugsweise hintereinander angeordnet, wobei die Zuleitungen Z₁ in Flussrichtung vor den Zuleitungen Z₂ liegen.

Der Abstand zwischen den Zuleitungen Z₁ und Z₂ beträgt vorzugsweise 10 bis 500%, besonders bevorzugt 50 bis 300%, ganz besonders bevorzugt 80 bis 200%, der Quadratwurzel des Ringleitungsquerschnitts Q.

Es sind vorzugsweise mindestens zwei Zuleitungen Z₁ und/oder Z₂ vorhanden, besonders bevorzugt zwei, drei, vier, fünf oder sechs Zuleitungen Z₁ bzw. Z₂, wobei die Zuleitungen Z₁ bzw. Z₂ vorzugsweise so angeordnet sind, dass sie eine gemeinsame Achse aufweisen (für zwei Zuleitungen Z₁ bzw. Z₂) oder einen symmetrischen Stern bilden (für mindestens drei Zuleitungen Z₁ bzw. Z₂) und die Achse bzw. Stern senkrecht zur Flussrichtung der neutralisierten Lösung befindet (Mehrfachzugabestellen).

Besonders vorteilhaft werden zwei, drei oder vier Mehrfachzugabestellen hintereinander angeordnet.

Beispielsweise können mindestens acht Zuleitungen Z₁ vorhanden sein, wobei jeweils vier Zuleitungen Z₁ kreuzförmig in die Ringleitung R münden, die mindestens 2 Vierergruppen an Zuleitungen Z₁ hintereinander und gegeneinander versetzt angeordnet sind.

Weiterhin kann mindestens eine dritte Zuleitung Z₃ in die Ringleitung R münden, wobei mindestens eine dritte Zuleitung Z₃ bedeutet, dass das Edukt 3, beispielsweise Wasser, über eine oder mehrere Zuleitungen Z₃ zugeführt wird, und die Zuleitung Z₃ in Flussrichtung vor der Zuleitung Z₁ liegt und/oder die Zuleitung Z₁ umhüllt.

Der Abstand zwischen den Zuleitungen Z₃ und Z₁ beträgt vorzugsweise 10 bis 500%, besonders bevorzugt 50 bis 300%, ganz besonders bevorzugt 80 bis 200%, der Quadratwurzel des Ringleitungsquerschnitts Q.

Vorzugsweise ist die Ringleitung R an mindestens einer Zuleitung Z₁ bis Z₃ als Venturi-Rohr ausgebildet.

Die Zuleitungen Z₁ bis Z₃ münden besonders bevorzugt alle in ein gemeinsames Venturi-Rohr.

Vorzugsweise ist die Ableitung Aₙ an mindestens einer Zuleitung Zₙ₁ bis Zₙ₃ als Venturi-Rohr ausgebildet.

Die Zuleitungen Zₙ₁ bis Zₙ₃ münden besonders bevorzugt alle in ein gemeinsames Venturi-Rohr.

## Patentansprüche

1. Neutralisationsverfahren, wobei mindestens eine ethylenisch ungesättigte Carbonsäure zumindest teilweise mit einer Base, wahlweise unter Zusatz von Wasser, vorneutralisiert wird, **dadurch gekennzeichnet, dass** die Vorneutralisation kontinuierlich durchgeführt wird, dass die vorneutralisierte Lösung teilweise in die Vorneutralisation rückgeführt wird und dass die vorneutralisierte Lösung in mindestens zwei Teillösungen aufgeteilt wird, wobei mindestens eine Teillösung nachbehandelt wird, so dass Teillösungen mit unterschiedlichem Neutralisationsgrad und/oder Feststoffgehalt entstehen, und wobei der Neutralisationsgrad der vorneutralisierten Lösung durch Zugabe von Base erhöht wird oder der Neutralisationsgrad der vorneutralisierten Lösung durch Zugabe von ethylenisch ungesättigter Carbonsäure gesenkt wird und/oder der Feststoffgehalt der vorneutralisierten Lösung durch Zugabe von Wasser gesenkt wird.

2. Verfahren gemäß Anspruch 1 , **dadurch gekennzeichnet, dass** die Nachbehandlung kontinuierlich durchgeführt wird.

3. Verfahren gemäß Anspruch 1 oder 2, **dadurch gekennzeichnet, dass** die Base wässriges Alkali ist.

4. Verfahren gemäß einem der Ansprüche 1 bis 3, **dadurch gekennzeichnet, dass** zwischen 25 und 95% der vorneutralisierten Lösung rückgeführt werden.

5. Verfahren gemäß einem der Ansprüche 1 bis 4, **dadurch gekennzeichnet**, die dass rückgeführte vorneutralisierte Lösung in der Vorneutralisation nacheinander mit Base und ethylenisch ungesättigter Carbonsäure versetzt wird.

6. Verfahren gemäß einem der Ansprüche 1 bis 4, **dadurch gekennzeichnet, dass** die rückgeführte vorneutralisierte Lösung in der Vorneutralisation nacheinander mit Wasser, Base und ethylenisch ungesättigter Carbonsäure versetzt wird.

7. Verfahren gemäß einem der Ansprüche 3 bis 6 **dadurch gekennzeichnet, dass** die ethylenisch ungesättigte Carbonsäure eine Temperatur von 15 bis 25°C und/oder das wässrige Alkali einen Alkaligehalt von weniger als 25 Gew.-% und eine Temperatur von 15 bis 30°C oder das wässrige Alkali einen Alkaligehalt von mindestens 25 Gew.-% und eine Temperatur von 30 bis 50°C und/oder falls eingesetzt das Wasser eine Temperatur von 15 bis 30°C aufweist.

8. Verfahren gemäß einem der Ansprüche 1 bis 7, **dadurch gekennzeichnet, dass** mindestens eine Dosierstelle für Base, ethylenisch ungesättigte Carbonsäure und wahlweise Wasser als Venturi-Rohr ausgebildet ist.

9. Verfahren gemäß einem der Ansprüche 3 bis 8, **dadurch gekennzeichnet, dass** das wässrige Alkali Natronlauge und/oder die ethylenisch ungesättigte Carbonsäure Acrylsäure ist.

10. Verfahren zur Herstellung wasserabsorbierender Polymere, umfassend ein Neutralisationsverfahren gemäß einem der Ansprüche 1 bis 9.

11. Vorrichtung zur kontinuierlichen Neutralisation, umfassend
i) eine Ringleitung R,
ii) mindestens eine erste Zuleitung Z₁ in die Ringleitung R,
iii) mindestens eine zweite Zuleitung Z₂ in die Ringleitung R,
iv) mindestens einen Wärmeaustauscher W in der Ringleitung R, wobei sich der Wärmeaustauscher W in Flussrichtung hinter den Zuleitungen Z₁ und Z₂ befindet,
v) mindestens zwei Ableitungen Aₙ aus der Ringleitung R, wobei sich die Ableitungen Aₙ in Flussrichtung hinter dem Wärmeaustauscher W befinden,
vi) mindestens eine Zuleitung ZXₙ₁ und/oder ZXₙ₃ in mindestens einer Ableitung Aₙ,
vii) eine Pumpe P und
viii) wahlweise einen Behälter B zwischen dem Wärmeaustauscher W und der Ableitungen Aₙ.

12. Vorrichtung gemäß Anspruch 11, **dadurch gekennzeichnet, dass** die Ringleitung R und/oder mindestens eine der Ableitungen Aₙ an der Einmündung mindestens einer Zuleitung Z₁, Z₂, ZXₙ₁ oder ZXₙ₃ als Venturi-Rohr ausgebildet ist.

## Claims

1. A neutralization process in which at least one ethylenically unsaturated carboxylic acid is at least partly preneutralized with a base, optionally with addition of water, which comprises conducting the preneutralization continuously, recycling the preneutralized solution partly into the preneutralization and dividing the preneutralized solution into at least two part-solutions, where at least one part-solution is aftertreated so as to give part-solutions having a different degree of neutralization and/or solids content, and where the degree of neutralization of the preneutralized solution is increased by adding base or the degree of neutralization of the preneutralized solution is lowered by adding ethylenically unsaturated carboxylic acid and/or the solids content of the preneutralized solution is lowered by adding water.

2. The process according to claim 1, wherein the aftertreatment is conducted continuously.

3. The process according to claim 1 or 2, wherein the base is aqueous alkali.

4. The process according to any of claims 1 to 3, wherein between 25 and 95% of the preneutralized solution is recycled.

5. The process according to any of claims 1 to 4, wherein the recycled preneutralized solution, in the preneutralization, is admixed successively with base and ethylenically unsaturated carboxylic acid.

6. The process according to any of claims 1 to 4, wherein the recycled preneutralized solution, in the preneutralization, is admixed successively with water, base and ethylenically unsaturated carboxylic acid.

7. The process according to any of claims 3 to 6, wherein the ethylenically unsaturated carboxylic acid has a temperature of from 15 to 25°C and/or the aqueous alkali has an alkali content of less than 25% by weight and a temperature of from 15 to 30°C or the aqueous alkali has an alkali content of at least 25% by weight and a temperature of from 30 to 50°C and/or, if used, the water has a temperature of from 15 to 30°C.

8. The process according to any of claims 1 to 7, wherein at least one metering point for base, ethylenically unsaturated carboxylic acid and, if desired, water is designed as a Venturi tube.

9. The process according to any of claims 3 to 8, wherein the aqueous alkali is sodium hydroxide solution and/or the ethylenically unsaturated carboxylic acid is acrylic acid.

10. A process for preparing water-absorbing polymers, comprising a neutralization process according to any of claims 1 to 9.

11. An apparatus for continuous neutralization, comprising
i) a ring line R,
ii) at least one first inlet Z₁ into the ring line R,
iii) at least one second inlet Z₂ into the ring line R,
iv) at least one heat exchanger W in the ring line R, the heat exchanger W being disposed beyond the inlets Z₁ and Z₂ in flow direction,
v) at least two outlets Aₙ from the ring line R, the outlets Aₙ being disposed beyond the heat exchanger W in flow direction,
vi) at least one inlet ZXₙ₁ and/or ZXₙ₃ in at least one outlet Aₙ,
vii) a pump P and
viii) if desired, a vessel B between the heat exchanger W and the outlets Aₙ.

12. The apparatus according to claim 11, wherein the ring line R and/or at least one of the outlets Aₙ at the entry of at least one inlet Z₁, Z₂, ZXₙ₁ or ZXₙ₃ is designed as a Venturi tube.

## Revendications

1. Procédé de neutralisation, au moins un acide carboxylique éthyléniquement insaturé étant préneutralisé au moins partiellement avec une base, éventuellement avec addition d'eau, **caractérisé en ce que** la pré-neutralisation est effectuée en continu, **en ce que** la solution pré-neutralisée est partiellement recyclée dans la pré-neutralisation et **en ce que** la solution pré-neutralisée est séparée en au moins deux solutions partielles, au moins une solution partielle étant traitée ultérieurement, de sorte que des solutions partielles se forment, qui présentent différents degrés de neutralisation et/ou différentes teneurs en solides, et le degré de neutralisation de la solution pré-neutralisée étant augmenté par l'addition de base ou le degré de neutralisation de la solution pré-neutralisée étant diminué par l'addition d'acide carboxylique éthyléniquement insaturé et/ou la teneur en solides de la solution pré-neutralisée étant diminuée par l'addition d'eau.

2. Procédé selon la revendication 1, **caractérisé en ce que** le traitement ultérieur est effectué en continu.

3. Procédé selon la revendication 1 ou 2, **caractérisé en ce que** la base est un alcali aqueux.

4. Procédé selon l'une quelconque des revendications 1 à 3, **caractérisé en ce qu'**on recycle entre 25 et 95% de la solution pré-neutralisée.

5. Procédé selon l'une quelconque des revendications 1 à 4, **caractérisé en ce que** la solution pré-neutralisée recyclée est mélangée dans la pré-neutralisation, consécutivement, avec la base et l'acide carboxylique éthyléniquement insaturé.

6. Procédé selon l'une quelconque des revendications 1 à 4, **caractérisé en ce que** la solution pré-neutralisée recyclée est mélangée dans la pré-neutralisation, consécutivement, avec de l'eau, la base et l'acide carboxylique éthyléniquement insaturé.

7. Procédé selon l'une quelconque des revendications 3 à 6, **caractérisé en ce que** l'acide carboxylique éthyléniquement insaturé présente une température de 15 à 25°C et/ou l'alcali aqueux présente une teneur alcaline inférieure à 25% en poids et une température de 15 à 30°C ou l'alcali aqueux présente une teneur alcaline d'au moins 25% en poids et une température de 30 à 50°C et/ou, si elle est utilisée, l'eau présente une température de 15 à 30°C.

8. Procédé selon l'une quelconque des revendications 1 à 7, **caractérisé en ce qu'**au moins un site de dosage pour la base, l'acide carboxylique éthyléniquement insaturé et le cas échéant l'eau est réalisé sous forme de tuyau Venturi.

9. Procédé selon l'une quelconque des revendications 3 à 8, **caractérisé en ce que** l'alcali aqueux est de la lessive de soude caustique et/ou l'acide carboxylique éthyléniquement insaturé est de l'acide acrylique.

10. Procédé pour la préparation de polymères absorbant l'eau, comprenant un procédé de neutralisation selon l'une quelconque des revendications 1 à 9.

11. Dispositif pour la neutralisation continue, comprenant
i) une conduite annulaire R,
ii) au moins une première conduite d'alimentation Z₁ dans la conduite annulaire R,
iii) au moins une deuxième conduite d'alimentation Z₂ dans la conduite annulaire R,
iv) au moins un échangeur thermique W dans la conduite annulaire R, l'échangeur thermique W se trouvant, dans le sens d'écoulement, en aval des conduites d'alimentation Z₁ et Z₂,
v) au moins deux dérivations Aₙ de la conduite annulaire R, les dérivations Aₙ se trouvant, dans le sens d'écoulement, en aval de l'échangeur thermique W,
vi) au moins une conduite d'alimentation ZXₙ₁ et/ou ZXₙ₃ dans au moins une dérivation Aₙ,
vii) une pompe P et
viii) éventuellement un récipient B entre l'échangeur thermique W et les dérivations Aₙ.

12. Dispositif selon la revendication 11, **caractérisé en ce que** la conduite annulaire R et/ou au moins une des dérivations Aₙ à l'entrée d'au moins une conduite d'alimentation Z₁, Z₂, ZXₙ₁ ou ZXₙ₃ est réalisée sous forme de tuyau Venturi.
